# EUROPEAN PATENT APPLICATION

(11) **EP 1 724 358 A1**
(43) Date of publication of application: **22.11.2006**
(21) Application number: 06425323.0
(22) Date of filing: 12.05.2006
(51) Int. Cl.: C12Q 1/46, C12Q 1/44

(54) **Diagnostic kit for Hirschsprung disease (congenital megacolon)**

(30) Priority: 16.05.2005 IT MI20050881
(71) Applicant: Martucciello, Giuseppe, 27100 Pavia (IT); Favre, Anna, 16132 Genova (IT)
(72) Inventor: Martucciello, Giuseppe, 27100 Pavia (IT); Favre, Anna, 16132 Genova (IT)
(74) Representative: Long, Giorgio

(57) **Abstract**

The present invention relates to a diagnostic kit for Hirschsprung disease comprising one or more appropriately stored, pre-measured histochemical enzymatic techniques. These techniques are selected from AChE, ANE, NADPH-d, SDH and LDH.

In a second aspect thereof, this invention also relates to a diagnostic method for determining Hirschsprung disease.

## Description

The present invention relates to a Hirschsprung disease diagnostic kit and a Hirschsprung disease diagnostic method for this disease.

Hirschsprung disease is a congenital abnormality characterised by an absence of nerve cell formation in a section of the intestine during foetal development. This abnormality occurs with a frequency of 1 in 5000 live births.

The intestinal nerve cells, also known as ganglion cells, are responsible for intestinal motility and for the movement of intestinal content.

In Hirschsprung disease the nerve cells are absent in all or part of the colon and rectum resulting in an inability to pass faecal material. The consequences are extremely dangerous and can cause intestinal obstruction and other complications including mortality.

The current treatment for this disease is a surgical operation where the malformed section of the intestine is removed and the remaining healthy section of the intestine is connected to the anal canal. After such operation, the patient can expect to live a normal, healthy life.

An early and accurate diagnosis of the disease is vital to ensure a favourable outcome and to avoid extremely dangerous consequences such as death.

Among the techniques available for diagnosis of the disease, with the biopsy a sample of rectal tissue is taken and the presence/absence of intestinal neurones, their morphology and their vital and metabolic capacity are determined in the laboratory.

Such analysis is normally carried out using appropriate techniques involving histochemical enzyme staining that reveals the presence or absence of specific enzymatic activities. This is followed by a morphological analysis of the tissue samples stained using the above technique under a microscope.

The most important histochemical enzyme stains are: acetylcholinesterase (AChE) to analyse the presence and distribution of colinergic nerve fibres with acetylcholinesterase activity that is notably increased when the disease is present; alpha-naphthylacetate esterase or non-specific esterase (ANE), NADPH diaphorase (NADPH-d), succinate dehydrogenase (SDH) and lactate dehydrogenase (LDH) that demonstrate the presence of submucosal ganglion cells, which are absent when the disease is present.

Each histochemical technique includes a certain number of reactive components, including a specific substrate for each enzyme, a colorant or a precipitant salt and a buffer. Currently, the reaction is prepared in the laboratory by mixing solutions of said reactive components in suitable solvents in the order indicated and applying the resulting solution to serial sections of the intestine biopsy. The reaction shows enzymatic activity present in the biopsy by producing coloration visible under microscope. A coloured precipitant is formed as a product of the reaction and its presence and intensity can be observed under the microscope.

Once prepared, the solution (medium) must be applied to the biopsy as soon as possible. In solution, the reactive components are unstable and their activity progressively degenerates over time; they are completely inactive one hour after mixing.

Consequently, a fresh medium is usually prepared every time it is necessary to carry out a diagnosis. Considering that preparing the different solutions making up the final medium can take a long time, e.g. 1 - 2 hours, it is clear that in the current state it is extremely difficult, if not impossible, to carry out a fast or urgent diagnosis.

Another problem linked to these diagnostic methods is the fact that all laboratories have their own procedures for applying these techniques and for conducting a diagnosis. Consequently, in the current state, a diagnosis carried out in different laboratories using the same technique and the same biological sample could result in different results.

This is all to the detriment of patients affected by this disease who cannot count on safe, accurate and most of all, quick diagnostic methods.

The problems reported above are resolved by a Hirschsprung disease diagnostic kit as outlined in the attached Claims.

In a first aspect, the present invention concerns a kit for the diagnosis of Hirschsprung disease including pre-measured reactive components for the application of AChE enzymatic technique, divided into at least three solutions.

The basic reactive components for AChE application technique are: an enzymatic substrate, a precipitant salt, a binding agent and a complexing agent, buffered at pH 5 to 7, preferably pH 5.6 to 6.3 or more preferably about pH 6.0 with a suitable buffer solution.

The kit according to the invention preferably also includes a reaction-diffusing blocking agent and a solvent.

For the application of the AChE technique, the kit's reactive components are divided into at least three solutions: the first is a buffer solution at pH 6.0 (medium A); the second solution includes a mixture of the enzymatic substrate, the precipitant salt and the complexing agent (medium B); the third solution is an aqueous solution of binding agent (medium C).

Preferably, medium B further includes a reaction-diffusion blocking agent, and a solvent, preferably water.

Medium B is prepared starting with aqueous solutions of the precipitant salt, complexing agent and reaction-diffusion blocking agent, in the concentrations shown in Table 1. Aliquots of said solutions are taken to have a molar ratio between the components as indicated in Table 1. The aliquots are mixed and the solid enzymatic substrate is added in the molar ratio with the other components as reported in Table 1. The solution is stirred until completely dissolved and is then diluted with water. The buffer solution (medium A) and the binding solution (medium C) can be prepared with the desired concentrations or purchased readymade (Table 1).

**Table 1**

| AChE technique | | | | |
|---|---|---|---|---|
| Reactive components | Solution concentration | Preferred solution concentration | Millimolar ratio | More preferred millimolar ratio |
| **Medium A** | | | | |
| Acetate buffer | 0.05-0.2 M | 0.1 M | | |

| **Medium B** | | | | |
|---|---|---|---|---|
| Acetylthiocholine iodide (enzymatic substrate) | | | 1 | 1 |
| Anhydrous copper sulphate (precipitant salt) | 20-70 mM | 60 mM | 1.3-3.0 preferably 1.5-2.0 | 1.7 |
| Anhydrous sodium citrate (complexing agent) | 0.05-0.5 M | 0.2 M | 2-4, preferably 2.5-3.5 | 2.9 |
| Iso-OMPA (tetra(monoisopropyl)pyrophospahtetetramide) (blocking agent for reaction diffusion) | 4-10 mM | 8 mM | 0.01-0.1, preferably 0.03-0.07 | 0.05 |
| H₂O (solvent) | | | | |

| **Medium C** | | | | |
|---|---|---|---|---|
| Potassium ferrocyanide (binding agent) | 2-7 mM | 5 mM | 0.05-0.4, preferably 0.15-0.35 | 0.29 |

The best option for selling the kit is through freeze-drying compound B.

During delivery, the kit may be kept at room temperature (solutions A and C in liquid form, solution B freeze-dried), however, the kit should be stored at +4°C to give the kit a 3 months shelf-life.

Therefore, the inventive kit preferably includes three solutions (A, B, C). Said solutions are preferably divided, in turn, in two or more aliquots of equal volume in suitable containers, such as vials, Eppendorf tubes, etc.

Preferably, each medium is divided into 10 to 30 equal aliquots, more preferably 18 to 25, all equal. Most preferably, each medium is divided into 20 aliquots of equal volume.

Medium B aliquots are freeze-dried, or medium B may be freeze-dried before it is divided into aliquots, while medium A and C aliquots are stored at room temperature. Alternatively, solutions A, B and C can be frozen separately at a temperature of -20°C to -4°C.

The laboratory technician who uses the kit will find each medium divided into an equal number of aliquots, stored in individual containers. The containers storing medium A aliquots will be advantageously marked with a same letter, for example A, while the containers storing medium B and C will be marked with other two letters, such as B and C, respectively.

Once the kit has been opened, the laboratory technician is tasked with mixing an aliquot of medium A with an aliquot of medium B and C, thus obtaining the final solution, applying it to cryostat sections of an intestinal biopsy. The sections will preferably have a thickness of 15 microns and will be frozen and incubated with the final solution at a suitable temperature and for a suitable time, preferably in a humid chamber.

To ease the laboratory technician's task and to speed up the analysis and diagnosis, the inventive kit can also advantageously include:
- one or more trays in which the biopsy and a substance used to freeze the tissue, for example OCT (optimal cutting temperature compound), a well known substance commonly used in pathology-anatomy laboratories commercially produced by Tissue Tek, are placed.
- one or more silanized slides used to prevent the cryostat biopsy sections detaching during the enzymatic reaction;
- an instruction manual for the application of the technique and for Hirschsprung disease diagnosis.

Incubation should occur at a temperature of 25 to 38°C, preferably at about 37°C for a period of 40 to 80 minutes, preferably for about 60 minutes.

During this time period at this temperature, a chemical reaction between the acetylcholinesterase enzyme that may be present in the biopsy and the reactive components in the kit's final solution will occur.

The acetylcholinesterase enzyme is a hydrolysing enzyme that hydrolyses the acetylcholine neurotransmitter producing acetate and choline at the neuronal level.

Acetylthiocholinesterase tissue enzymatic activity is revealed through the hydrolysation of the enzymatic substrate, acetylcholine iodide, by the enzyme present in the neuronal cells (both in the neurosomes and in the axonal and dendritic outgrowth) forming thiocholine that binds to the copper ions present in the medium. The copper-thiocholine complex is thus formed, which precipitates in the reaction in a brown colour. The copper ions detach from the copper sulphate salt in the presence of potassium cyanide.

The copper ions form a complex with sodium citrate. The Iso-OMPA works as an inhibitor of the non-specific esterase and as a reaction stabiliser. The laboratory technician thus places the biopsy under a microscope and the pathological-anatomist carries out the Hirschsprung disease diagnosis.

In the absence of ganglion cells of the enteric nervous system (Hirschsprung disease or Congenital Megacolon), the AChE technique will show using coloration, the increase of positive acetylcholinesterase fibres of extraneous origin. These dramatically increase in the presence of the disease, but they are almost completely absent in a healthy rectum.

In a second aspect, the kit includes the necessary reactive components for the application of a further enzymatic histochemical technique: alpha-naphthylacetate esterase or non-specific esterase (ANE).

This technique comprises an enzymatic substrate (alpha-naphthylacetate), and a colorant pararosaniline hexazonium, buffered at pH 6.8 to 7.8, preferably pH 7.25, which are pre-measured in the molar ratios indicated in Table 2.

Said components can either be mixed to give a single solution that can be freeze-dried or frozen at -20°C to - 4°C or they can be divided into four solutions and stored, preferably at room temperature.

Pararosaniline hexazonium solution is prepared by dissolving pararosaniline hydrochloride in water then adding 37% hydrochloric acid and heating and stirring the mix until it is completely dissolved. When the solution has cooled, it is filtered and stored in a fridge at about 4°C for six months or more (solution 1). Separately, a sodium nitrite aqueous solution (solution 2) is prepared. An equal volume of solution 1 and solution 2 is mixed to form the pararosaniline hexazonium solution (quaternary ammonium salt).

The alpha-naphylacetate solution and the buffer at the concentrations indicated in table 2 are also prepared. These solutions can be mixed in the molar concentrations indicated in table 2 in order to obtain a single solution that will be then freeze-dried or frozen. Alternatively, a solution of alpha-naphylacetate, sodium nitrite, pararosaniline hydrochloride and phosphate buffer is prepared, respectively, which are included separately in the kit and are preferably stored at room temperature.

**Table 2**

| ANE Technique | | | | |
|---|---|---|---|---|
| Reactive components | Solution concentration | Preferred solution concentration | Millimolar ratio | Preferred millimolar ratio |
| Alpha-naphthylacetate (enzymatic substrate) | 0.5-3% by weight | 1% by weight | 1 | 1 |
| Sodium nitrite (starting material for the colorant) | 1-6% by weight | 4% by weight | 30-65, preferably 45-60 | 52.7 |
| Pararosaniline hydrochloride (starting material for the colorant) | 90-150 mM | 124 mM | 5-15 preferably 8-12 | 11.3 |
| Dibasic sodium phosphate (system buffer component) | 0.05-0.25 M | 0.15 M | | |
| Monobasic sodium phosphate (system buffer component) | 0.05-0.25 M | 0.15 M | | |

The mixture of all components is placed in a suitable container, which is advantageously a different colour than the colour of the AChE technique containers. This mixture can be freeze-dried or frozen. Preferably, the mixture is freeze-dried. Alternatively, the four said solutions are put in four containers that are advantageously marked with the letters A, B, C and D in a colour that is different to the colour of the AChE technique containers.

The technician who uses the kit will find the ANE technique components pre-measured and mixed, or divided into four solutions. Therefore, the technician needs only apply the mixture to the biopsy or mix the four pre-measured solutions to obtain the mixture to be applied to the biopsy. After application, an incubation at a temperature of 20°C to 37°C is carried out, preferably at about 25°C for a time period of 4 to 10 minutes, preferably 5 to 10 minutes.

Alpha-naphthylesterase or non-specific esterase activity is caused by a set of hydrolase enzymes that have the characteristics of hydrolysing, in the alpha position, short-chain fatty acids. Such enzymes are present in the intestinal sub-mucosal ganglion cells, which are known to be absent in the last section of the colon in the presence of Hirschsprung disease.

If ganglion cells are present in the biopsy, the esterase tissue enzymes hydrolyse the alpha-naphylacetate, which frees the naphthol that can then bind with the diazonium salts (hexazotized pararosaniline) and produce an insoluble red brown precipitant at around pH 7.5.

Because this method involves extremely short reaction times, it can be applied on the mucosal/submucosal rectal biopsy or on the seromuscular intestinal biopsy taken in different segments of the intestine during the surgical colon resection operation. Thereby, diagnostic information about the length of the section to be removed can be provided during the operation.

In the third aspect, the inventive kit further comprises the necessary reactive components for the application of a further enzymatic histochemical technique: the NADPH-diaphorase technique. This technique includes an enzymatic substrate, a colorant-precipitant agent and an enzyme activator, buffered at pH 6.8 to 7.0, preferably at around pH 7.4, divided into at least two solutions.

The first solution is a mixture of aqueous solutions, at suitable concentrations, of the colorant-precipitant agent and the activator enzyme, buffered at pH 6.8 to 7.0, preferably at about pH 7.4, and diluted with a suitable quantity of water (medium A). The second solution is an aqueous solution of the enzymatic substrate (medium B). The components of the NADPH-diaphorase technique are mixed in the molar ratios indicated in Table 3. The concentrations of the solutions of the various components are also reported.

**Table 3**

| NADPH-diaphorase technique | | | | |
|---|---|---|---|---|
| Reactive components | Solution concentration | Preferred solution concentration | Millimolar ratios | Preferred millimolar ratios |
| **Medium A** | | | | |
| Nitro-BT Nitro-BT (nitro blue tetrazonium chloride) (colorant-precipitant agent) | 3-7 mM | 5 mM | 0.2-0.5, preferably 0.3-0.47 | 0.46 |
| TRIS buffer | 0.05-0.3M | 0.2M | | |
| MgCl₂ (enzyme activator) | 1-7 mM | 5 mM | 0.05-0.3 preferably 0.1-0.25 | 0.19 |
| H₂O | | | | |

| **Medium B** | | | | |
|---|---|---|---|---|
| NADPH (enzymatic substrate) | 15-35 mM | 27 mM | 1 | 1 |

Mediums A and B are advantageously kept in different-coloured containers to the other enzymatic techniques containers, and each container is marked with a letter, for example, A and B. These mediums can be freeze-dried or frozen from -20°C to -4°C.

The laboratory technician shall then proceed by mixing medium A with medium B, thus obtaining the final medium that will be applied to the biopsy, keeping them incubated at a temperature of 28°C to 40°C, preferably at about 37°C for a period of 10 to 15 minutes, preferably 7 to 10 minutes.

The NADPH-diaphorase enzyme is present both in the ganglion cells, which are absent in the presence of disease, and in the fibres belonging to the non-adrenergic - non-colinergic (NANC) system that result in reduced activity localised in the mitochondria cells in the presence of disease.

If normally present in the biopsy, the enzyme hydrolyses the NADPH substrate producing electrons that reduce the tetrazolium salts (Nitro-BT). The colorant that was clear and soluble before the reduction is transformed into a blue precipitant that is localised in the mitochondria. The neurones are especially rich in these organelles both in the neurosomes and in the axonic outgrowths, which thus makes possible both localization and a functional assessment. This is carried out by observing colour intensity with a microscope.

This technique, like ANE technique, can be applied both on the mucosal/submucosal rectal biopsy or on the seromuscular intestinal biopsy taken from different segments of the intestine during the surgical colon resection operation, thus enabling the user to determine the length of the diseased section to be removed, either by itself, or preferably in combination with ANE technique.

In a fourth aspect, the inventive kit comprises the reactive components of a fourth enzymatic histochemical technique: the succinate dehydrogenase (SDH) technique.

This technique comprises an enzymatic substrate, a colorant-precipitant agent, buffered at pH 7 to 8, preferably about 7.6. Preferably, these reactive components are mixed to give a single reactive mixture that can be freeze-dried or frozen.

The reactive solution is a mixture of aqueous solutions at suitable concentrations of the buffered (pH about 7.6) colorant-precipitant agent and enzymatic substrate.

The colorant-precipitant agent used for the application of this technique is either Nitro-BT or MTT Thiazolyl Blue (Thiazolyl blue Tetrazolium Bromide.) If the second colorant is used, it should be used in combination with a metallic salt solution.

Accordingly, if the Nitro-BT colorant is used, the components should be mixed in the molar ratios indicated in Table 4 whereas if the MTT Thiazolyl Blue colorant is used, the components should be mixed in the molar ratios indicated in Table 4-bis.

**Table 4**

| SDH Technique | | | | |
|---|---|---|---|---|
| Reactive components | Solution concentration | Preferred solution concentration | Molar ratios | Preferred molar ratios |
| Nitro-BT (colorant-precipitant agent) | 0.5-1.5 mM | 1.15 mM | 0.005-0.015 preferably 0.008-0.013 | 0.011 |
| Phosphate buffer | 0.05-0.3 M | 0.2 M | | |
| Sodium succinate (enzymatic substrate) | 0.1-0.3 M | 0.2 M 1 | | 1 |

**Table 4-bis**

| SDH Technique | | | | |
|---|---|---|---|---|
| Reactive components | Solution concentration | Preferred solution concentration | Molar ratios | Preferred molar ratios |
| MTT Thiazolyl Blue (colorant-precipitant agent) | 1-3.0 mM | 2.41 mM | 0.01-0.06 preferably 0.02-0.05 | 0.03 |
| Cobalt chloride (metallic salt) | 1-4% by weight | 2% by weight | 0.03-0.1, preferably 0.06-0.09 | 0.08 |
| Tris-HCl buffer | 0.05-0.3M | 0.2 M | | |
| Sodium succinate (enzymatic substrate) | 0.1-0.3 M | 0.2 M | 1 | 1 |

The kit mixture is applied to the biopsy and incubated at a temperature of 35°C to 40°C, preferably at about 38°C, for a period of 10 to 60 minutes, preferably about 20 minutes.

The dehydrogenase succinate enzyme is present in mature ganglion of the enteric nervous system and thus its presence enables the cells' maturation to be examined. In Hirschsprung disease, in fact, ganglion cells can be completely absent, partially present or can be present at a state of maturation that is not sufficient for normal intestinal activity.

If present in the biopsy, succinate dehydrogenase enzyme hydrolyses the sodium succinate substrate producing electrons that reduce the tetrazolium salts (Nitro-BT). The colorant that was clear and soluble before the reduction is transformed into a blue precipitant that is localised in the mitochondria. The neurones are especially rich in these organelles in the neurosomes, which makes possible the localisation and a functional assessment of the same. If the SDH technique is used with the formulation shown in table 4-bis, the yellow-coloured MTT Thiazolyl Blue is rapidly reduced to formazan by means of the electrons resulting from the hydrolysis of the sodium succinate substrate. The formazan is a dark blue insoluble product that binds to cobalt as it precipitates, forming a complex having an extreme colour intensity.

In a fifth aspect, the inventive kit also includes the necessary reactive components for lactate dehydrogenase (LDH) technique application, said components being mixed to form a single solution that is stable over time if stored in a suitable manner, for example, by freezing or freeze-drying. Alternatively, said components can be divided into at least two parts (A and B). In this case, the first part (medium A) comprises a mixture of aqueous solutions at suitable concentrations of the enzymatic substrate, colorant-precipitant agent and of enzyme activator, buffered at pH 6.5 to 7.2, preferably about pH 6.8 (medium A). The second part comprises the blocking agent of the reaction diffusion in solid form (compound B). The components of medium A and the compound B are mixed in the molar ratios indicated in table 5.

**Table 5**

| Lactate dehydrogenase technique | | | | |
|---|---|---|---|---|
| Reactive components | Solution concentration | Preferred solution concentration | Molar ratios | Preferred molar ratios |
| **Medium** | | | | |
| Sodium L-lactate (enzymatic substrate) | 0.5-2M | 1M | 1 | 1 |
| Beta-NAD | 0.05-0.5 M | 0.1M | 0.05-0.8, preferably 0.08-0.5 | 0.1 |
| Nitro-BT (colorant-precipitant agent) | 0.05-0.3% by weight | 0.1% by weight | 0.001-0.01, preferably 0.002-0.006 | 0.003 |
| Phosphate buffer | 0.1-0.2 M | 0.15 M | | |
| MgCl₂.6H₂O (enzymatic activator) | 0.01-0.1 M | 0.05 M | 0.01-1, preferably 0.03-0.08 | 0.05 |

| **Compound B** | | | | |
|---|---|---|---|---|
| Polyvinyl pyrrolidone K25 (blocking agent of reaction diffusion) | | | 0.005-0.02, preferably 0.008-0.017 | 0.0145 |

Medium A can be stored at room temperature or freeze-dried or frozen. Having opened the kit, the laboratory technician will find medium A and B advantageously kept in two different-coloured containers to the containers of the other histochemical techniques and marked with a different letter, for example, A and B. In this case, the technician must bring medium A up to a volume of 40 ml with water and then add compound B.

Once the final medium is obtained, it is then applied to the biopsy.

The lactate dehydrogenase enzyme is present in the ganglion cells' mitochondria, which are absent in the presence of the disease and hydrolyses the sodium L-lactate substrate thereby producing electrons that reduce the tetrazonium salts (Nitro-BT). The colorant that was clear and soluble before the reduction is transformed into a blue precipitant that is localised in the mitochondria.

Magnesium chloride is an enzymatic activator and polyvinyl pyrrolidone K25 is a polyvinyl alcohol that fixes the coloured precipitant on the section thus preventing the diffusion of the same.

The neurones are especially rich in these organelles in the neurosomes, which thus makes possible localisation and a functional assessment of the same.

This technique is applied to a mucosal-sub-mucosal rectum biopsy and is incubated at a temperature of 25°C to 40°C, preferably at about 37°C for 10 to 30 minutes, preferably for about 20 minutes.

The inventive kit comprises at least one of said histochemical techniques, preferably at least two. Preferably, if the kit includes a single technique, this is the AChE technique; if it contains two techniques, they are AChE and ANE.

Alternatively, one to five individual kits can be arranged, each one comprising one single enzymatic technique.

In a further aspect thereof, the present invention concerns a pre-operation diagnosis method for Hirschsprung disease or Congenital Megacolon, comprising the following steps:
a) Taking a biopsy from a patient;
b) Slicing the biopsy into cryostat serial sections with a preferred thickness of 15 micron;
c) Applying at least three of the histochemical techniques contained in the kit to the sections of each biopsy; each 15 micron section can be treated only with a single technique;
d) Using a microscope to view the obtained results;
e) Combining the results obtained with each technique to determine the final diagnosis.

The rationale to the invention's diagnosis method is that one technique alone, although important, like acetylcholinesterase (AChE), can never be conclusive for the diagnosis, nor can it cover both the requirements of pre-operation diagnosis (studying mucosal-sub-mucosal rectum biopsies) and mid-operation (studying seromuscular biopsies taken from different intestine segments to determine the length of affected section to be removed).

The invention's diagnostic method therefore enables results obtained from the application of at least three histochemical techniques, preferably at least four histochemical techniques, or more preferably five histochemical techniques to be combined. Said at least three techniques are AChE, ANE and NADPH-diaphorase. Each of these techniques can highlight different aspects in the enteric nervous system, thus keeping the risks of false positives and false negatives both in the pre-operation phase and during the mid-operation diagnosis to a minimum. Trials with at least three combined techniques has surprisingly demonstrated that the synergy and the complimentarity of the results negates the possibility of error.

For the mid-operation diagnosis, at least two seromuscular intestinal biopsies are taken, during the operation, from different points along the intestine that are then cut in two serial sections and frozen.

ANE and NADPH diaphorase techniques are applied on to two sections of each biopsy, respectively. They are left to react for the periods of time and temperatures as indicated above, and then the colorations are observed with a microscope and the results are compared.

In the mid-operation diagnosis, the ANE and NADPH-d diagnosis are used as their reaction times can be measured in just minutes. Consequently, while the patient undergoes the operation, these techniques can quickly identify the length of the affected section that needs to be removed.

In particular, the functions of each technique and their synergistic effects can be summarised as follows:
**Acetylcholinesterase (AChE):** in the absence of ganglion cells from the enteric nervous system (Hirschsprung disease or Congenital Megacolon) AChE technique will detect the increase of positive acetylcholinesterase fibre of extraneous origin. These dramatically increase in the presence of disease, but are almost completely absent in a healthy rectum. It is applied on a mucosal biopsy from the rectum. It is not used to determine and locate the sub-mucosal ganglion (as mucosal fibres could be mistaken for sub-mucosal ganglion, thus risking a false negative result). A further limitation of this technique is that it cannot be used for a mid-operation examination due to the length of incubation time. This technique is also not able to determine the exact extent of the disease.
**Non-specific esterase (ANE)**: out of all the techniques tested here, this one is best for locating ganglion cells both for a sub-mucosal rectum biopsy and for an sero-muscular intestinal biopsy during mid-operation diagnosis. This technique complements the first technique because it does not locate the nerve fibres, but instead stains the ganglion neurons of an intense red-brown colour. The incubation time makes it suitable for mid-operation diagnosis.
**Succinate dehydrogenase (SDH)**: only locates very well ganglion from a mature enteric nervous system, thus can be used in association with other techniques and it is able to determine the degree of maturation of the intestinal innervation. It does not locate the nerve fibres.
**NADPH-Diaphorase (NADPH-d):** this technique is able to distinguish both ganglion cells and fibres of the NANC (non-adrenergic, non-colinergic) system. It can be used in both pre-operation and mid-operation phases. This technique is able to recognise the absence of ganglion cells (Hirschsprung disease aganglia) and can assess a deficiency in the nitrergic system that innervates the intestinal walls.
**Lactate dehydrogenase (LDH)**: Lactate dehydrogenase colours the sub-mucosal ganglion of the pre-operation rectum biopsy with an intense blue.

An example of how to carry out a combination of pre-operation results is shown in Table 6. Each technique is applied to different serial sections of the same biopsy.

As shown by the table, if AChE technique is applied when conditions are normal and no disease is present, no colour will be observed; however, if ANE, NADPH-d, LDH and SDH techniques are used, the appearance of their respective colours will be observed.

Conversely, if an intense brown coloration occurs after the AChE technique is applied and no colour shows, after other techniques are applied, this would enable a confident diagnosis of the disease to be made.

**Table 6**

| | AChE | ANE | NADPH-d | LDH | SDH |
|---|---|---|---|---|---|
| Absence of disease | - | + red-brown sub-mucosal ganglion | + blue sub-mucosal ganglion | + blue sub-mucosal ganglion | + blue sub-mucosal ganglion only if mature |
| pathology present (Hirschsprung disease) | +++ dramatic increase of blue nerve fibres in the mucous membrane | - | - | - | - |

In the mid-operation diagnosis, each biopsy is cut into two sections and each section is treated with just one technique (ANE or NADPH-d).
The diagnosis is negative if a red-brown coloration is observed with the microscope when using ANE technique and blue if using NADPH-d technique. Conversely, the diagnosis is positive if said colorations are not observed, i.e. when the myenteric plexus ganglion are absent.

### EXAMPLES

Some examples of how the kit is used are reported herein below. The histochemical techniques can be comprised in a single kit or there can be a plurality of kits each containing just one enzymatic technique.

| **AChE Technique** | | |
|---|---|---|
| **components** | **solution in ml** | **Number of Millimoles** |
| **Medium A** | | |
| a) Acetate buffer 0.1 M pH 6 | 180 ml | |

| **Medium B** | | |
|---|---|---|
| b) Anhydrous sodium citrate 0.2 M in water | 5 ml | 1 |
| c) Anhydrous copper sulphate 60mM in water | 10 ml | 0.6 |
| d) Iso-OMPA 8mM in water | 2 ml | 0.016 |
| e) Acetylcholine iodide (solid) | 100 mg | 0.35 |
| H₂O | 10 ml | |

| **Medium C** | | |
|---|---|---|
| f) Potassium ferrocyanide 5mM in water | 20 ml | 0.1 |

The aqueous solutions a), b), c), d) and f) are prepared in the concentrations indicated in the tables. Solutions b), c) and d), 100mg acetylcholine iodide and 10 ml water are mixed under stirring until the acetylcholine is completely dissolved to form medium B.

Medium A (180 ml) is divided into 20 aliquots of 9 ml each, which are stored in 20 vials that are marked with the letter A.

Medium B (27 ml) is divided into 20 aliquots of 1.35 ml each that are likewise stored in 10 ml vials, marked with the letter B. Each aliquot is subsequently freeze-dried.

Medium C is divided into 20 aliquots of 1 ml each that are likewise stored in 10 ml vials, marked with the letter C.

Upon use, 9 ml medium A are poured into a vial of freeze-dried medium B and 1 ml solution C is added. Thereby, 20 packs of 10 ml AChE ready to use are obtained.

| **ANE Technique** | | |
|---|---|---|
| **components** | **ml of solution** | **Number of Millimoles** |
| a) alpha-naphylacetate 1% in acetone | 0.6 ml | 0.011 |
| b) pararosaniline hexazonium | 2 ml | |
| c) monobasic sodium phosphate 0.15 M | 25 ml | |
| e) dibasic sodium phosphate 0.15 M in water | 100 ml+X | |

Solutions a), c) and e) are prepared in the concentrations indicated in the table. The solution b) is prepared by dissolving 1 g pararosaniline hydrochloride in 20 ml distilled water, 5 ml fuming hydrochloric acid (37%) are added, then heated under stirring. The solution is left to cool and then filtered; this solution can be stored in the fridge at 4°C in the dark for 6 six months or more.

Separately, a 4% aqueous solution of nitrite sodium is prepared. 1 ml (0.124 mmols) of pararosaniline hydrochloride solution is added to 1 ml (0.58 mmols) sodium nitrate thus obtaining the pararosaniline hexazonium diazonium salt, which is the colorant to be used. To obtain the final medium, 2 ml of this last solution are mixed with 0.6 ml alpha-naphylacetate, 25 ml monobasic sodium phosphate and 100 ml dibasic sodium phosphate are added and carrying on the addition until a pH of 7.25 is reached. The X in the previous table shows the exact quantity necessary to get to pH 7.25.

| **NADPH-diaphorase technique** | | |
|---|---|---|
| **components** | **ml of solution** | **Number of Millimoles** |
| **Medium A** | | |
| a)Nitro-BT 4 mg/ml (aqueous solution) | 2.5 ml | 0.0115 |
| b) TRIS buffer 0.2 M pH 7.4 | 2.5 ml | |
| c) MgCl₂ 5 mM in water | 1 ml | 0.005 |
| H₂O | 3 ml | |

| **Medium B** | | |
|---|---|---|
| d) NADPH 20 mg/ml (aqueous solution) | 1 ml | 0.027 |

Solutions a), b), c) and d) are prepared at the concentrations indicated. Solutions a), b) and c) are mixed in the amounts indicated and 3 ml water is added, thus obtaining medium A.

Medium A and B can be separately stored, frozen or freeze-dried, for long periods without losing their activity.

Having opened the kit, the technician will mix medium B to medium A to obtain the final medium.

| **Succinate dehydrogenase (SDH) technique- example 1** | | |
|---|---|---|
| **components** | **ml of solution** | **Number of millimoles** |
| a) sodium succinate 0.2 M in water | 10 ml | 2 |
| b) phosphate buffer 0.2 M pH 7.6 | 10 ml | 2 |
| c) Nitro-BT 1 mg/ml in water | 20 ml | 0.023 |

| **Succinate dehydrogenase (SDH) technique- example 2** | | |
|---|---|---|
| **components** | **ml of solution** | **Number of millimoles** |
| a) sodium succinate 0.2 M in water | 4.0 ml | 0.8 |
| b) Tris-HCl buffer 0.2 M pH 7.4. | 3.4 ml | 0.68 |
| c) MTT Thiazolyl Blue | 10 mg | 0.03 |
| d) cobalt chloride in H₂O | 2% by weight | 0.062 |
| e) distilled water | 2.2 ml | |

Solutions made up of components in example 1 are prepared in the concentrations indicated. The solutions are mixed in the quantities indicated to obtain the medium present in the kit. The medium is stable when frozen or freeze-dried without losing its activity. In example 2, solutions a), b) and d) are prepared and mixed in the aliquots indicated, water is added and then the MTT Thiazolyl Blue, thereby obtaining the final medium for applying on to the biopsy.

| **Lactate dehydrogenase (LDH) technique:** | | |
|---|---|---|
| **components** | **ml of solution** | **Number of millimoles** |
| **Medium A** | | |
| a) sodium L-lactate 1 M in water | 4 ml | 4 |
| b) Beta-NAD 0.1 M in phosphate buffer 0.15 M | 4 ml | 0.4 |
| c) MgCl₂.6H₂O 0.05 M in water | 4 ml | 0.2 |
| d) phosphate buffer 0.15 M pH 6.8 | 10 ml | 1.5 |
| e) Nitro-BT 0.1% in water | 10 ml | 0.0115 |

| **Medium B** | | |
|---|---|---|
| f) polyvinyl pyrrolidone K25 1.4g | | 1.45 |

Solutions a), b), c), d) and e) are prepared in the concentrations indicated above and are mixed in the above amounts to give medium A. Medium B is made up of 1.4 g of polyvinyl pyrrolidone K25. The medium is stable when frozen or freeze-dried. Having opened the kit, the technician must bring medium A up to a volume of 40 ml with distilled water and then add 1.4 g of polyvinyl pyrrolidone, stirring the solution until complete dissolution is obtained.

In a further aspect thereof, the invention concerns a kit for Hirschsprung disease including the pre-measured components for the application of at least one histochemical enzymatic technique selected from: ACHE, ANE, NADPH-diaphorase, SDH and lactate dehydrogenase.

Furthermore, the invention also concerns the application of the diagnostic kit for abnormalities of the adult enteric nervous system.

### PRE-OPERATION DIAGNOSIS

The mucosal-submucosal rectum biopsy is placed in the plastic tray that is contained in the kit and is frozen using OCT slowly immersed into liquid nitrogen.

After freezing, the biopsy is cut into five cryostat sections, each one with a thickness of 15 micron and placed on the silanized slide contained in the kit.

Five kits are opened, each one containing an enzymatic technique, AChE, ANE, NADPH-diaphorase, SDH and LDH, or a single kit containing three or more techniques.

The final mediums for each technique are prepared as explained above and are applied to the cryostat sections, one for each section.

This is followed with incubation at the following temperatures and for the following time periods, at the end of which coloured precipitates will form that are visible with a microscope:

| Technique | Incubation time | Incubation temperature | Precipitant colour |
|---|---|---|---|
| AChE | 1 hour | 37°C | brown |
| ANE | 6 minutes | Room temperature | red-brown |
| NADPH-diaphorase | 10 minutes | 37°C | blue |
| SDH | 20 minutes | 38°C | blue |
| LDH | 20 minutes | 37°C | blue |

A microscope is used to observe the presence or absence of the coloured enzymatic activities and their intensities. If the results are examined in an integrated way (as explained above) a secure Hirschsprung disease diagnosis can be made.

### MID-OPERATION DIAGNOSIS

During the operation to remove the affected intestine part, the surgeon needs to know exactly the extent of the disease to avoid removing a healthy section of intestine or leaving an aganglion section behind. For this reason, the surgeon takes more samples of different intestinal segments. For example, biopsies are taken from the sigmoid rectum, the descending colon and the transverse colon, etc.

Each biopsy is placed in an appropriate container, comprised in the kit, is frozen and is cut into two or more sections. Each section is placed on a silanized slide and incubated with the final medium with ANE or NADPH-diaphorase technique, prepared as described above. To the purpose, two kits containing each ANE or NADPH-d technique, respectively, or a single kit containing both techniques can be used. After 6 and 8 minutes respectively, the coloration can be observed with a microscope. By repeating this operation for all biopsies, the extent of the disease can be determined. An example of the diagnosis is shown in table 7.

**Table 7**

| | ANE | NADPH-d |
|---|---|---|
| Absence of disease | + in red-brown of the myenteric ganglion | + in blue of the myenteric ganglion |
| Presence of Hirschsprung disease | - | - |

### ADVANTAGES

Using the kit and the pre-measured reactive components for histoenzymatic techniques considerably reduces analysis time, and can accommodate even urgent diagnosis. It substantially eases the work of the histopathological technician who needs only open the kit, mix the pre-measured mediums and apply the obtained solutions to the biopsy. Thus error margins are reduced to a minimum.

Furthermore, the use of pre-measured components and the standardisation of the analysis procedure and diagnosis allows secure and uniform results to be obtained from different laboratories. The kit also makes it possible to apply up to five histochemical techniques on the same biopsy that has been divided into cryostat sections, thus examining different aspects of the disease. By cross-referencing results, a diagnosis can be obtained that is extremely accurate, with obvious advantages for patients' health.

Using the kit is also extremely advantageous for mid-operation diagnosis as it enables a fast and accurate diagnosis, thus eliminating the possibility of removing a healthy section of intestine or conversely, leaving an affected section of intestine behind.

A further advantage is that the reaction time for AChE technique is reduced from 90 to 60 minutes with freeze-drying.

## Claims

1. A diagnosis kit for Hirschsprung disease comprising the pre-measured reactive components for the application of the AChE enzymatic technique, said components being: acetylthiocholine iodide, a buffer at pH 5.8 - 6.2, a precipitant salt, a binding agent, a reaction-diffusing blocking agent and a solvent **characterised in that** said components are divided into at least three solutions.

2. The kit according to Claim 1 where said at least three solutions comprise respectively: an aqueous buffer solution at pH 5.8 to 6.2 (medium A); a mixture of acetylthiocholine iodide, the precipitant salt, the complexing agent, the reaction-diffusing blocking agent and the solvent (medium B); an aqueous solution of the binding agent(medium C).

3. The kit according to Claims 1 or 2, wherein said acetylcholine iodide, precipitant salt, complexing agent, reaction-diffusing blocking agent and said binding agent are comprised in the following molar ratios respectively: 1 : 1.3-3.0 : 2-4 : 0.01-0.1 : 0.05-0.4, preferably 1 : 1.5-2.0 : 2.5-3.5 : 0.03-0.07 : 0.15-0.35, preferably 1 : 1.7 : 2.9 : 0.05 : 0.29.

4. The kit according to any Claim 1 to 3, wherein said buffer solution is an acetate buffer solution with a concentration of 0.05 to 0.2 M, preferably about 0.1 M; said precipitant salt is an aqueous solution of anhydrous copper sulphate having a concentration of 20 to 70 mM, preferably about 60 mM; said complexing agent is an aqueous solution of anhydrous sodium citrate with a concentration of 0.05 to 0.5 M, preferably about 0.2 M; said reaction-diffusing blocking agent is Iso-OMPA; said binding agent is an aqueous solution of potassium ferrocyanide with a concentration of 2 to 7 mM, preferably about 5 mM.

5. The kit according to any Claim 1 to 4 which is stored by freezing, or said mediums A and C are stored in solution form at room temperature or said medium B is freeze-dried.

6. The kit according to any Claim 1 to 5, wherein said mediums A, B and C are divided into aliquots of equal amounts.

7. The kit according to any Claim 1 to 6 further comprising the pre-measured reactive components for the application of the histochemical enzymatic ANE technique, said components being the enzymatic substrate alpha-naphylacetate and a colorant buffered at pH 6.8 to 7.8.

8. The kit according to Claim 7 wherein said enzymatic substrate is a solution of alpha-naphylacetate in acetone with a concentration of 0.5 to 3%, preferably about 1%; said colorant is pararosaniline hexazonium obtained by mixing an equal volume of sodium nitrite solution with a concentration of 1 to 6%, preferably 4%, and pararosaniline hydrochloride with a concentration of 90 to 150 mmols, preferably about 120 mmols; said buffer comprises an aqueous solution of monobasic sodium phosphate and an aqueous solution of dibasic sodium phosphate both having a concentration of 0.05 to 0.25 M, preferably about 0.15 M.

9. The kit according to Claim 7 or 8 wherein the molar ratio between alpha-naphylacetate, pararosaniline hydrochloride and sodium nitrite is, respectively: 1 : 30-65 : 2-15, preferably 1 : 45-60 : 8-12, more preferably 1:52,7:11,3.

10. The kit according to any Claim 7 to 9 wherein said components are mixed to give a single solution stored by freeze-drying or are divided in four solutions stored at room temperature.

11. A kit for Hirschsprung disease including only the pre-measured reactive components for the application of the ANE technique according to any Claim 7 to 10.

12. The kit according to any Claim 1 to 10 further comprising the pre-measured reactive components for the histochemical enzymatic NADPH-diaphorase technique, said components being the enzymatic substrate NADPH, a colorant-precipitant agent, an enzyme activator and a solvent, buffered at pH 6.8 to 7.0 wherein said components are divided into at least two solutions (medium A and B).

13. The kit according to Claim 12 wherein said at least two solutions comprise respectively: a mixture of colorant-precipitant agent, enzyme activator and solvent buffered at pH 6.8 to 7.0 (medium A); and an aqueous solution of NADPH (medium B).

14. The kit according to Claim 12 or 13 wherein said NADPH, colorant-precipitant agent and enzyme activator are included in the following molar ratios, respectively: 1 : 0.2-0.5 : 0.05-0.3, preferably 1 : 0.3-0.45 : 0.1-0.25, more preferably 1 : 0.43 : 0.19.

15. The kit according to any Claim 12 to 14 wherein said buffer is a TRIS buffer having a concentration of 0.05 to 0.3 M, preferably about 0.2 M; said enzymatic substrate is an aqueous solution of NADPH having a concentration of 15 to 35 mM, preferably about 27 mM; said colorant-precipitant agent is an aqueous solution of Nitro-BT having a concentration of 3 to 7 M, preferably about 4.6 mM; said enzyme activator is a MgCl₂ aqueous solution with a concentration of 1 to 7 mM, preferably 5 mM.

16. The kit according to any Claim 12 to 15 that is stored by freezing.

17. The kit according to any Claim 12 to 16 wherein said mediums A and B are stored in solution form at room temperature or by freeze-drying.

18. A kit for Hirschsprung disease comprising only the pre-measured reactive components for the application of the NADPH-diaphorase technique according to any Claim 12 to 17.

19. The kit according to any Claim 1 to 17 further comprising the pre-measured reactive components for the application of the histochemical enzymatic SDH technique, said components being the enzymatic substrate sodium succinate and a colorant-precipitant agent, buffered at pH 7 to 8.0 wherein said components are mixed to give a single solution.

20. The kit according to Claim 19 wherein said sodium succinate and colorant-precipitant agent are comprised in the following molar ratios, respectively: 1 : 0.005-0.015, preferably 1 : 0.008-0.013, more preferably 1 : 0.011.

21. The kit according to Claim 19 or 20 wherein said buffer is a phosphate buffer with a concentration of 0.05 to 0.3 M, preferably about 0.2 M; said enzymatic substrate is an aqueous solution of sodium succinate with a concentration of 0.1 to 0.3 M, preferably about 0.2M; said colorant-precipitant agent is a Nitro-BT aqueous solution with a concentration of 0.5 to 1.5 mM, preferably about 1.15 mM.

22. The kit according to Claim 19 further comprising a metallic salt, preferably an aqueous solution of cobalt chloride at 2% by weight.

23. The kit according to Claim 19 and 22 wherein said buffer is a Tris-HCl buffer with a concentration of 0.05 to 0.3 M, preferably about 0.2 M; said enzymatic substrate is a sodium succinate aqueous solution with a concentration of 0.1 to 0.3 M, preferably about 0.2M; said colorant-precipitant agent is MTT Thiazolyl Blue with a concentration in the final medium of 1 to 3.0 mM, preferably about 2.4 mM.

24. The kit according to Claims 19 and 22 wherein said sodium succinate, colorant-precipitant agent and metallic salt are comprised in the following molar ratios, respectively: 1 : 0.01-0.06 : 0.03-0.1, preferably 1 : 0.02-0.05 : 0.06-0.09, more preferably 1 : 0.03 : 0.08.

25. The kit according to any Claim 19 to 24 that is stored by freezing.

26. The kit according to any Claim 19 to 24 wherein said mixture is stored in solution form at room temperature or by freeze-drying.

27. A kit for Hirschsprung disease comprising only the pre-measured reactive components for the application of the SDH technique according to any Claim 19 to 26.

28. The kit according to any Claim 1 to 26 further comprising the pre-measured reactive components for the application of the histochemical enzymatic LDH technique, said components being a first enzymatic substrate sodium L-lactate, a second enzymatic substrate Beta-NAD, a colorant-precipitant agent, an enzyme activator and a reaction-diffusing blocking agent, buffered at pH 6.5 to 7.2 wherein said components are mixed to give a single solution that is either freeze-dried or frozen, or are divided into at least 2 parts (A and B).

29. The kit according to Claim 28 wherein said at least two parts include respectively: sodium L-lactate, Beta-NAD, the colorant-precipitant agent, the enzyme activator, buffered at pH 6.5 to 7.2 (medium A); the reaction-diffusing blocking agent (compound B).

30. The kit according to Claim 28 or 29 wherein said sodium L-lactate, Beta-NAD, colorant-precipitant agent enzyme activator and reaction-diffusing blocking agent are comprised in the following molar ratios, respectively: 1 : 0.05-0.8 : 0.001-0.01 : 0.01-1 : 0.005-0.02, preferably 1 : 0.08-0.5 : 0.002-0.006 : 0.03-0.08 : 0.008-0.17, more preferably 1 : 0.1 : 0.003-0.05 : 0.0145.

31. The kit according to one of Claims 28 to 30, wherein said buffer is a phosphate buffer solution with a concentration of 0.1 to 0.2 M, preferably about 0.15 M; said enzymatic substrate is a sodium L-lactate aqueous solution with a concentration of 0.5 to 2 M, preferably about 1 M; said second enzymatic substrate is a solution of Beta-NAD in phosphate buffer with a concentration of 0.05 to 0.5 mM, preferably about 0.1 mM; said colorant-precipitant agent is an aqueous solutions of Nitro-BT with a concentration of 0.05 to 3%, preferably about 0.1%; said enzyme activator is a MgCl.6H₂O aqueous solution with a concentration of 0.01 to 0.1M, preferably about 0.05M; said reaction-diffusing blocking agent is polyvinyl pyrrolidone K25.

32. The kit according to any Claim 28 to 31 that is stored by freezing.

33. The kit according to any Claim 28 to 32 wherein said medium A is stored as a solution at room temperature or by freeze-drying.

34. The kit for Hirschsprung disease comprising only the pre-measured reactive components for the application of the LDH technique according to any Claim 28 to 33.

35. The kit for Hirschsprung disease comprising at least one of the histochemical enzymatic techniques selected from: AChE, ANE, NADPH-diaphorase, SDH and LDH.

36. Use of the kit according to any Claim 1 to 35 for the diagnosis of abnormalities in the adult enteric nervous system.

37. A pre-operation diagnostic method for Hirschsprung disease diagnosis or ganglial Congenital Megacolon, comprising the following steps:
a. Taking a biopsy of a patient's intestine.
b. Dividing this biopsy into serial sections;
c. Applying the final medium of at least three of the histochemical techniques contained in the kit to the different sections according any Claim 1 to 35; each section is treated with only one technique;
d. Observing the obtained results with a microscope;
e. Matching the obtained results for each technique to determine the final diagnosis.

38. The method according to Claim 37 wherein said at least three enzymatic histochemical techniques are AChE, ANE and NADPH-diaphorase.

39. The method according to Claim 37 or 38 wherein between step c) and step d), there is a further incubation step at the following temperatures and time periods:
AChE 25-38°C, preferably about 37°C for 40-80 minutes, preferably about 60 minutes;
ANE 20-37°C, preferably about 25° C for 4-10 minutes, preferably about 6 minutes;
NADPH-diaphorase 28-40°C, preferably 37°C for 5-15 minutes, preferably about 8 minutes;

40. The method according to Claim 39 wherein if the SDH and LDH techniques are used, incubation occurs at the following temperatures and for the following time periods:
SDH 35-40°C, preferably about 38°C for 10-60 minutes, preferably about 20 minutes;
LDH 25-40° C, preferably about 37° C for 10-30 minutes, preferably about 20 minutes;

41. A mid-operation diagnostic method for Hirschsprung or ganglial Congenital Megacolon disease diagnosis, comprising the following steps:
a) Taking at least two sero-muscular intestinal biopsies from a patient during the operation; divide said biopsies into serial sections;
b) Applying the final medium of the histochemical ANE and NADPH-diaphorase techniques contained in the kit to each section according to any Claim 1 to 35 ; each section is treated with only one technique;
c) Incubating at a temperature of 20-37° C, preferably about 25° C for 4 to 10 minutes, preferably about 6 minutes, for the ANE technique and 28-40° C, preferably about 37°C for 5-15 minutes, preferably about 8 minutes, for the NADPH-diaphorase technique;
d) Observing the obtained results with a microscope;
e) Matching the results obtained for each technique to determine the final diagnosis.
